(19) **Europäisches Patentamt / European Patent Office / Office européen des brevets**

(11) **EP 2 965 690 A1**

(12) **EUROPEAN PATENT APPLICATION**
published in accordance with Art. 153(4) EPC

(43) Date of publication:
**13.01.2016 Bulletin 2016/02**

(51) Int Cl.:
***A61B 5/0476*** (2006.01)

(21) Application number: **14759461.8**

(22) Date of filing: **03.03.2014**

(86) International application number:
**PCT/JP2014/055230**

(87) International publication number:
**WO 2014/136704 (12.09.2014 Gazette 2014/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **04.03.2013 JP 2013042198**

(71) Applicant: **Brain Functions Laboratory, Inc. Kanagawa 230-0046 (JP)**

(72) Inventor: **MUSHA, Toshimitsu Yokohama-shi Kanagawa 230-0046 (JP)**

(74) Representative: **Calderbank, Thomas Roger et al Mewburn Ellis LLP City Tower 40 Basinghall Street London EC2V 5DE (GB)**

(54) **BRAIN FUNCTION ACTIVITY EVALUATION DEVICE AND EVALUATION SYSTEM USING SAME**

(57) Discrete Fourier transform is performed on an output of each of brain potential sensors, which measure a brain potential of a subject, for each of segments in order to obtain a discrete Fourier coefficient that has a frequency component, which is an integral multiple of a fundamental frequency that is an inverse number of a predetermined time width, within a predetermined frequency band. A mean value of squares of absolute values of Fourier coefficients is obtained. The Fourier coefficients are normalized using the mean value of the squares of the absolute values thereof in order to obtain a normalized power spectrum (NPS;j,m) that is a first parameter. A product of mean values of squares of absolute values of Fourier coefficients of adjoining frequency components in all the segments is normalized using a square value of the sum of the mean values of the adjoining frequency components in order to obtain a normalized power ratio (NPV;j,m) that is a second parameter. Two markers sNAT;j,m and vNAT;j,m are derived from the first and second parameters respectively in order to evaluate a brain function activity level.

FIG. 2

EP 2 965 690 A1

**Description**

BACKGROUND OF THE INVENTION

Field of the Invention

The present invention relates to a device that evaluates a brain function activity level by measuring the brain function activity level and an evaluation system using the device. In particular, the present invention is concerned with a device that evaluates the state of a brain function activity so as to discriminate a brain disease such as senile cognitive impairment, and a system using the device.

Description of the Related Art

Among dementias, Alzheimer's dementia (hereinafter AD) whose morbidity rises along with progress of aging has the cause thereof left unrevealed and the therapeutic method thereof left unestablished. In contrast, the number of AD patients is increasing. Therefore, not only in Japan but also in other countries in the world, a ratio by which increasing medical expenses and care expenses, which are needed to address AD, occupy a national budget is gradually augmenting. The augmentation has become a socially serious problem. As for a prophylactic approach, prophylaxis cannot help depending on early recognition and rehabilitation for activating the brain function. For example, in Japan, the population of elderly persons who are sixty-five years of age or older has reached thirty million. A diagnostic modality that assists in deciding whether an elderly person suffers from dementia is getting more significant. For prophylactic diagnosis aiming at the elderly, development of a novel diagnostic modality satisfying seven conditions listed below is expected.

(A) Inexpensive
(B) Noninvasive in that cerebrospinal fluid or blood is not collected
(C) Not accompanied by radiation exposure
(D) Highly sensitive
(E) Highly reliable
(F) Having the capability to present information permitting any physician other than a specialist of dementias to make diagnosis
(G) Operation of the device does not require an expertise.

A device that measures a brain function activity and satisfies the above conditions is needed. Brain function activity measuring devices based on scalp potential analysis which are devised by the present inventor are disclosed in Patent Documents 1 and 2 (Japanese Patent No. 4145344 and Japanese Patent No. 5118230). In these inventions, a scalp potential (hereinafter, a brain potential) is recorded using plural sensors, and brain potential components ranging from 2 Hz to 40 Hz are divided into frequency bands. A normalized power variance (hereinafter, NPV) is obtained from each of the frequency bands. A set of these variables is used as a marker to characterize an activity of encephalic neurons and gliacytes or the like (generically referred to as a brain function activity). A region in which a state of an activity is abnormal compared with a mean value of a group of normal controls is obtained. A set of newly obtained NPVs of a subject is compared with templates representing characteristics of respective diseases in order to quantize likelihoods to the brain function diseases.

The aforesaid existing arts are unsatisfactory from the viewpoints described below. The description below is based on findings acquired for the first time as a result of a large-scale clinical test.

(A) A quantity called the normalized power variance (NPV) of a brain potential does not provide information that is unique enough to characterize the brain function activity. A degree of discrimination between different diseases, for example, between Alzheimer's dementia and depression is not high.
(B) The marker includes an unnecessary offset that degrades sensitivity. A marker devoid of such an offset has to be introduced.
(C) A state specified by the marker is represented by a vector in a multidimensional space. A likelihood between states is represented by a cosine or inner product of an angle formed by vectors representing the states. The variable is not fully adaptable as the likelihood, and the meaning of the variable is not clarified.
(D) A recorded potential signal includes an artifact component. Since the artifact component becomes a factor that degrades sensitivity in separating diseases, there is no rigorous criterion to be used to determine a frequency band, which is an object of analysis, and a segment length of the recorded potential signal.

SUMMARY OF THE INVENTION

An object of the present invention is to provide a brain function activity level evaluation device and brain function activity level evaluation system capable of highly precisely discriminating different diseases from one another and displaying the discrimination between diseases.

In the present Description, some values are denoted by NPV;j,m, NLc,jm, or any other reference sign having a half-width additional character appended thereto. Whether the additional character is a superscript or subscript is requested to be decided by referencing an associated formula or drawing. In the present Description, what is referred to as a brain function activity is a brain activity caused by a brain function, and what is referred to as a brain function activity level is a degree of the brain function activity.

In order to solve the aforesaid problems, a brain function activity level evaluation device of the present invention includes plural sensors that are mounted on the head of a subject in order to measure a brain potential of the subject. The brain function activity level evaluation device further includes arithmetic means that: divides a brain potential, which is outputted from each of the sensors, into segments, which have a predetermined time width, on a time base; performs discrete Fourier transform for each of the segments so as to obtain a discrete Fourier coefficient that has a frequency component, which is an integral multiple of a fundamental frequency that is an inverse number of the predetermined time width, within a predetermined frequency band; obtains a mean value of squares of absolute values of the Fourier coefficients in all the segments, normalizes the obtained mean value of the squares of the absolute values of the Fourier coefficients so as to obtain a normalized power spectrum (NPS;j,m) that is a first parameter; and normalizes a product of mean values of squares of absolute values of Fourier coefficients of adjoining frequency components in all the segments using the square of the sum of the mean values of the adjoining frequency components so as to obtain a normalized power ratio (NPV;j,m) that is a second parameter. The first parameter and the second parameter are used to evaluate a brain function activity level.

Further, as an embodiment of the brain function activity level evaluation device of the present invention, the arithmetic means obtains a first marker sNAT;j,m and a second marker vNAT;j,m by subtracting a mean value of values of the frequency component, which are derived from all the sensors, in each of the normalized power spectrum (NPS;j,m) that is the first parameter, and the normalized power ratio (NPV;j,m) that is the second parameter. Each of the markers is characterized by a position in a multidimensional sNAT space or vNAT space in which sub-markers determined with the number of sensors and the number of discrete frequencies respectively are expressed.

As another embodiment of the brain function activity level evaluation device of the present invention, the arithmetic means calculates an sZ score (sZ;j,m;x:NLc) using the first marker (sNAT;x,jm) relevant to a subject x, a template state (sNAT;NLc,jm) of normal controls that is a mean value of the first markers obtained in advance from a predetermined group of normal controls in the same manner as the aforementioned one, and a standard deviation thereof (sσ;NLc,jm). The arithmetic means further calculates a vZ score (vZ;j,m;x:NLc) using the state of the subject x (vNAT;x,jm) determined with the second marker, a template state (vNAT;NLc,jm) determined with a mean value of the second markers obtained in advance in the same manner from the predetermined group of normal controls, and a standard deviation thereof (vσ;NLc,jm). The arithmetic means visualizes or displays the state of the activity of the brain at an associated position in a brain surface image on the basis of the values of the sZ score (sZ;j,m;x:NLc) and vZ score (vZ;j,m;x:NLc). As a function of a normalized distance (for example, Maharanobis distance) between a template state (for example, a mean state of a group of numerous AD patients), which characterizes any of various cerebral diseases, and the state of the subject, likelihoods of the subject to the disease are determined. By expressing the likelihoods with the shortness of the normalized distance, the meaning of the likelihoods becomes apparent, and separation between different diseases can be displayed.

A brain function activity level evaluation system as another embodiment of the present invention includes at least a brain function activity measuring terminal including plural sensors that are mounted on the head of a subject in order to measure a brain potential of the subject, an interface via which the brain potential outputted from each of the sensors is transmitted to outside, and an arithmetic unit, and a calculation center connected to the brain function activity measuring terminal over a communication line. The calculation center is characterized in that:

(1) the calculation center includes arithmetic means that divides a brain potential, which is outputted from each of the sensors and sent from the brain function activity measuring terminal, into segments, which have a predetermined time width, on a time base, that performs discrete Fourier transform for each of the segments so as to obtain a Fourier coefficient that has a frequency component, which is an integral multiple of a fundamental frequency that is an inverse number of the predetermined time width, within a predetermined frequency band, that obtains a mean value of squares of absolute values of the Fourier coefficients in all the segments, that normalizes the obtained mean value of the squares of the absolute values of the Fourier coefficients so as to obtain a normalized power spectrum (NPS;j,m) that is a first parameter, that normalizes a product of mean values of squares of absolute values of Fourier coefficients of adjoining discretized frequency components using a square of the sum of the mean values of the adjoining frequency components so as to obtain a normalized power ratio (NPV;j,m) that is a second parameter; and

(2) the calculation center transmits the obtained first parameter (NPS;j,m) and second parameter (NPV;j,m) to the brain function activity measuring terminal.

Further, in a brain function activity level evaluation system as another embodiment of the present invention, the arithmetic means of the calculation center obtains a first marker and a second marker by subtracting a mean value of values of the frequency component, which are derived from all the sensors, in each of the normalized power spectrum that is the first parameter, and the normalized power ratio that is the second parameter.

Further, in a brain function activity level evaluation system as another embodiment of the present invention, the arithmetic means of the calculation center calculates an sZ score using the value of the first marker relevant to a subject, a template state regarded as a mean value of the first markers obtained in advance in the same manner from a predetermined group of normal controls, and a standard deviation thereof, and further calculates a vZ score using the value of the second marker relevant to the subject, a template state regarded as a mean value of the second markers obtained in advance from the predetermined group of normal controls, and a standard deviation thereof. The brain function activity measuring terminal includes a unit that receives the calculated sZ score and vZ score over the communication line, and visualizes or displays a brain function activity level at associated positions in a brain surface image on the basis of the sZ score and vZ score.

Further, in a brain function activity level evaluation system as another embodiment of the present invention, the arithmetic means of the calculation center calculates likelihoods of the subject to the template state of the group of normal controls on the basis of the calculated sZ score and vZ score, uses the likelihoods to calculate difference likelihoods signifying to which of the template state of the group of normal controls and a template state of a group of AD patients the subject is more similar, and visualizes or displays the state of a brain function activity of the subject.

A program that is another embodiment of the present invention allows a computer to execute a procedure of: dividing a brain potential, which is outputted from each of plural sensors that are mounted on the head of a subject in order to measure the brain potential of the subject, into segments, which have a predetermined time width, on a time base; performing discrete Fourier transform for each of the segments so as to obtain a discrete Fourier coefficient that has a frequency component, which is an integral multiple of a fundamental frequency that is an inverse number of the predetermined time width, within a predetermined frequency band; obtaining a mean value of squares of absolute values of the Fourier coefficients in all the segments; normalizing the obtained mean value of the squares of the absolute values of the discrete Fourier coefficients so as to obtain a normalized power spectrum (NPS;j,m) that is a first marker; normalizing a product of mean values of squares of absolute values of discrete Fourier coefficients of adjoining frequency components using a square value of the sum of the mean values of the adjoining frequency components so as to obtain a normalized power ratio (NPV;j,m) that is a second marker; obtaining differences obtained by subtracting a mean value of values of the frequency component, which are derived from all the sensors, from the values of the frequency component in each of the normalized power spectrum that is the first parameter, and the normalized power ratio that is the second parameter, and obtaining a first marker (sNAT;j,m) and second marker (vNAT;j,m) by removing the offset values.

Advantageous Effects of Invention

In the present invention, a power spectrum of a brain potential is normalized with a mean value of a power spectrum within a given frequency band in order to obtain a normalized power spectrum NPS. A recorded brain potential signal is divided into segments, and an inverse number of a segment length is regarded as a fundamental frequency. A power distribution with respect to a frequency that is a positive integral multiple of the fundamental frequency is used as a raw material of a first parameter. As a second parameter, a normalized power ratio NPV obtained by normalizing a ratio of discretized power spectral components with a mean power of the powers of the spectral components is adopted. The parameters NPS and NPV are obtained by performing normalization on a frequency axis. A mean value of values of the frequency component derived from all channels is subtracted from the values of the frequency component in each of the parameters NPS and NPV, whereby a zero level is reset. This results in two markers sNAT and vNAT.

A state in which mean values (sZ;j,m;x:NLc)m and (vZ;j,m;x:NLc)m of Z scores of the two markers with respect to a frequency m are positive or negative is referred to as a hyperactive and under-synchronous state in which a brain function activity associated with the markers sNAT and vNAT is hyperactive and under-synchronous. A state in which the mean values are smaller is referred to as a hypoactive and over-synchronous state. As a result, the state of the brain function activity can be classified into four combinational states of a hyperactive and over-synchronous state, hyperactive and under-synchronous state, hypoactive and over-synchronous state, and hypoactive and under-synchronous state. The brain function activity can be characterized in more detail.

Accordingly, a distance between a template AD state, which is a mean of a group of numerous patients suffering from a certain disease, for example, AD patients, and a subject state is represented by a normalized distance expressed with quotients by standard deviations of the template AD state. Likelihoods are expressed based on the shortness of the distance. Accordingly, the meaning of the likelihoods becomes apparent and separation between diseases can be achieved.

According to the present invention, two newly introduced markers are used to display an abnormal region, in which a brain function activity is abnormal, on a brain surface in more detail. Various brain diseases can be detected in an early stage, and likelihoods to a template state characterizing any of the various brain diseases can be quantized. Different brain diseases can be highly precisely identified and discriminated from a normal control state. The states of the brain diseases can be imaged and displayed on a standard brain surface. Therefore, brain disease diagnosis can be realized inexpensively, noninvasively, highly sensitively, and highly reliably. This modality is expected to prevail in medium- and small-scale medical institutions.

BRIEF DESCRIPTION OF THE DRAWINGS

Fig. 1 is a block diagram showing a configuration of a brain function activity level evaluation device that is an example of the present invention;
Fig. 2 is a flowchart describing processing of the brain function activity level evaluation device of the present invention;
Fig. 3 is a diagram showing sensitivity/specificity curves with respect to a difference likelihood;
Fig. 4A is a diagram showing t values in a t-test indicating significance of state separation and being concerned with sNAT;j,m;
Fig. 4B is a diagram showing the t values in the t-test indicating the significance of state separation and being concerned with vNAT;j,m;
Fig. 5 is a likelihood graph based on a pair of difference likelihoods;
Fig. 6A is a diagram showing an example of display of NAT images and having vZ;j;x:AD allocated to associated positions on a standard brain surface;
Fig. 6B is a diagram showing an example of display of the NAT images and having sZ;j;x:AD allocated to associated positions on the standard brain surface;
Fig. 6C is a diagram showing an example of display of SPECT images;
Fig. 7 is a block diagram of a brain function activity level evaluation system that is another example of the present invention; and
Fig. 8 is a flowchart describing processing of the brain function activity level evaluation system of the present invention.

DETAILED DESCRIPTION OF THE PREFERRED EMBODIMENTS

Examples will be described below in conjunction with the drawings.

Example 1

(1) Hardware configuration of a brain function activity level evaluation device

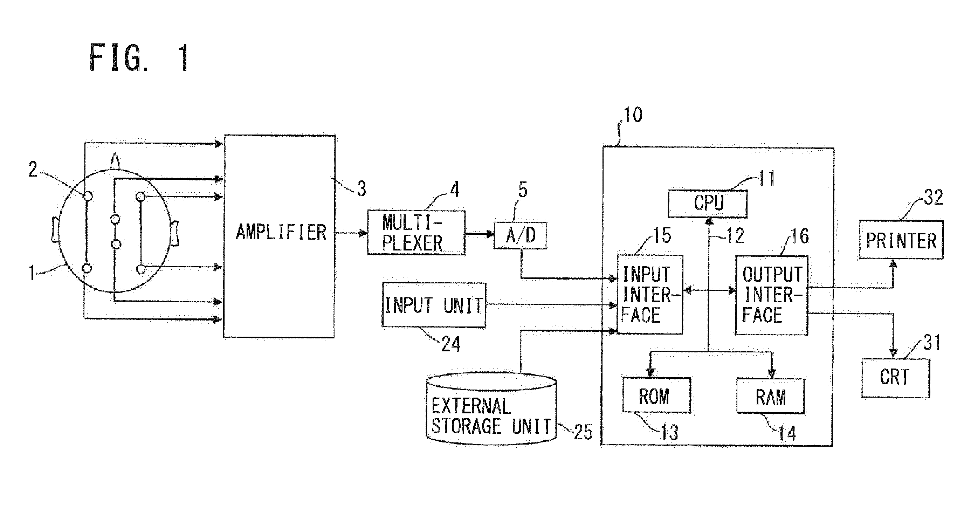

Fig. 1 is a block diagram showing a configuration of a brain function activity level evaluation device that is an example of the present invention. The brain function activity level evaluation device that is an example of the present invention includes brain potential sensors or magnetoencephalographic sensors (which may be called electrodes and may hereinafter be generically called sensors) 2, an amplifier 3 that amplifies a brain potential measured by each of the sensors 2, a multiplexer 4, an analog-to-digital converter (A/D converter) 5, a computer 10, an input unit 24 such as a keyboard, an external storage unit 25 in which programs and others are stored, a display unit 31 such as a CRT, and a printer 32. The computer 10 includes an interface (I/F) 15 via which digitized measured brain potential data is inputted, a CPU 11, a ROM 13, a RAM 14, an output interface (I/F) 16, and a bus 12 over which the components are interconnected.

The ROM 13 is a read-only storage medium, and the RAM 14 is a memory in which brain potential data sent from the input unit 24 such as a keyboard or the A/D converter 5 is stored during computation. The CPU 11 is an arithmetic unit that reads a program out of the external storage unit 25 or ROM 13, and performs various computations on the brain potential data sent from the A/D converter 5 and read from the RAM 14. The results of computation are displayed on the display unit 31 (CRT) via the output interface 16. The printer 32 prints out data or a waveform displayed on the display unit 31. The external storage unit 25 may not be used but the programs and others may all be stored in advance in the ROM 13.

The sensors 2 are formed with plural electrodes, for example, about twenty-one electrodes, and mounted on the head 1 in order to measure a brain potential based on a brain function activity. Otherwise, a cap or helmet in which the about twenty-one electrodes are included in advance may be mounted on the head 1 in order to measure the brain potential. Needless to say, any technique other than the use of the cap or helmet may be adopted as long as it can measure the brain potential based on the brain function activity. In this case, the sensors 2 are disposed at positions stipulated in or determined in conformity with the International 10-20 Standard, and a sensor (not shown) is disposed at, for example, the right earlobe regarded as the position of a reference potential. The brain potential measured by the sensor 2 is fed to the analog-to-digital converter (A/D converter) 5 via the amplifier 3 and multiplexer 4. Digitized measured brain potential data is fed to the computer 10 via the input interface (I/F) 15. The measured brain potential data may be passed through the input interface 15 as it is. Alternatively, only components falling within a pre-defined frequency band (for example, a predetermined frequency band wider than the frequency band of alpha waves) may be subjected to digital filtering processing and then outputted.

The brain function activity level evaluation device may be configured as a stand-alone device. When the brain function activity level evaluation device is configured as the stand-alone device, a physician can immediately obtain diagnosis assistive information even at a clinical site in an isolated island in which an Internet environment is unavailable. As far as the Internet environment is available, more appropriate diagnosis assistance can be attained over the Internet using the external storage unit as a server.

(2) Arithmetic processing of the brain function activity level evaluation device

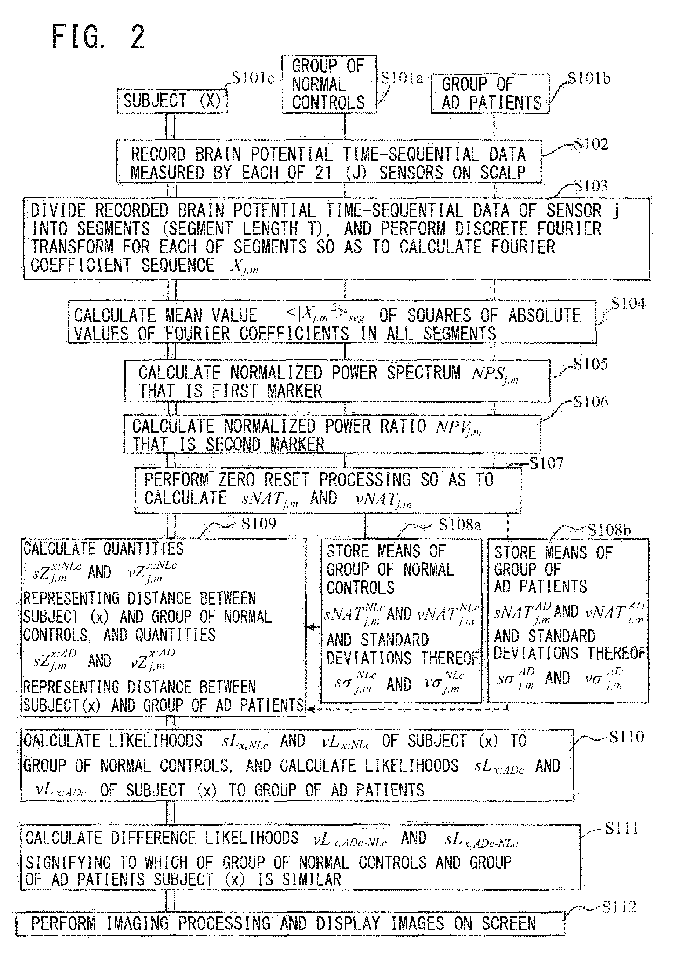

Fig. 2 is a flowchart describing processing of the brain function activity level evaluation device that is an example of the present invention. In the example of the present invention, the processing of the brain function activity level evaluation device is performed on each of a subject, a group of normal controls, and a group of AD patients.

To begin with, production of a database concerning the group of normal controls will be described (processing begins at S101c). A group of normal controls including a predetermined number of persons is determined in advance by conducting the mini mental state examination (MMSE), the magnetic resonance imaging (MRI) for image examination, the single photon emission computing tomography (SPECT), or the like which is an existing method intended to evaluate a cognitive function. Each of the persons has time-sequential data of a brain potential (scalp potential) thereof measured using each of the twenty-one (J) sensors 2, and has the data recorded (step S102). A potential signal of each of the sensors 2 is sampled at intervals of 5 ms. As for a frequency range, specific frequency portions (for example, predetermined frequencies ranging from 4 Hz to 20 Hz) are extracted through bandpass filter processing. The optimal frequency band ranging from 4 Hz to 20 Hz will be described later.

Recorded brain potential time-sequential data obtained by the sensor j (electrode) (j ranges from 1 to 21) is divided into segments (segment length T) on a time base. The brain potential is subjected to discrete Fourier transform for each of the segments in order to obtain a Fourier coefficient $X_{j,m}$ that has a frequency component $mf_0$ which is an integral m multiple of a fundamental frequency fo (=1/T) relevant to each segment (step S103). Thereafter, a mean value $\langle |X_{j,m}|^2 \rangle_{seg}$ of squares of absolute values of the Fourier coefficients in all the segments is calculated (step S104). The mean value $\langle |X_{j,m}|^2 \rangle_{seg}$ is referred to as a power of the frequency component.

A normalized power spectrum NPS;j,m that is a first parameter is calculated as a value normalized with the mean value $\langle |X_{j,m}|^2 \rangle_{seg}$ according to a formula (1) below (step S105).

[Math. 1]

$$NPS_{j,m} = \left\langle \left| X_{j,m} \right|^2 \right\rangle_{seg} \Big/ \left\langle \left\langle \left| X_{j,m} \right|^2 \right\rangle_{seg} \right\rangle_m$$

The normalized power spectrum NPS;j,m represents an average distribution of the powers of the frequency component $mf_0$ on the channel j. The calculated NPS;j,m is stored in the RAM 14.

In order to characterize a power ratio between adjoining frequency components, a normalized power ratio NPV;j,m that is a second parameter is calculated as a dimensionless quantity according to a formula (2) below (step S106). The calculated NPV;j,m is stored in the RAM 14.

[Math. 2]

$$NPV_{j,m} = 4 \left\langle \left| X_{j,m} \right|^2 \right\rangle_{seg} \left\langle \left| X_{j,m+1} \right|^2 \right\rangle_{seg} \Big/ \left\{ \left\langle \left| X_{j,m} \right|^2 \right\rangle_{seg} + \left\langle \left| X_{j,m+1} \right|^2 \right\rangle_{seg} \right\}^2$$

The power ratio p;j,m between adjoining frequency components is expressed by a formula (3) below.

$$[\text{Math. 3}]$$

$$p_{j,m} = \left\langle \left| X_{j,m-1} \right|^2 \right\rangle_{seg} \Big/ \left\langle \left| X_{j,m} \right|^2 \right\rangle_{seg}$$

By assigning the formula (3) to the formula (2), a formula (4) below is obtained.

$$[\text{Math. 4}]$$

$$NPV_{j,m} = \frac{4 p_{j,m}}{\left\{ 1 + p_{j,m} \right\}^2}$$

According to the formula (4), when p;j,m=1, NPV;j,m takes on a maximum value of 1. This represents the power ratio between frequency components that adjoin on a frequency axis, and signifies a gradient of a power spectrum of a signal whose intensity is modulated and which is fed to the brain due to a brain function activity. That is, when p;j,m=1, an average power ratio is 1. The brain function activity becomes random and signal transmission is not carried out.

Zero reset processing is performed on the parameters in order to calculate sNAT;j,m and vNAT;j,m (step S107). More particularly, in order to enhance a spatial distribution on the channel j with respect to the frequency mf0 in each of the normalized power spectrum NPS;j,m and normalized power ratio NPV;j,m, offset values appearing in the spatial distribution have to be removed because they weaken a relative change concerning the space. Markers sNAT and vNAT are obtained by removing the offset values according to formulae (5) and (6) below. Accordingly, sensitivity is improved.

$$[\text{Math. 5}]$$

$$sNAT_{j,m} = \left\langle NPS_{j,m} \right\rangle_{seg} - \left\langle NPS_{j,m} \right\rangle_{seg,m},$$

$$[\text{Math. 6}]$$

$$vNAT_{j,m} = \left\langle NPV_{j,m} \right\rangle_{seg} - \left\langle NPV_{j,m} \right\rangle_{seg,m}$$

The aforesaid steps S102 to S107 are repeatedly performed on all normal controls, whereby group means for the group of normal controls, <sNAT;NLc,jm> and <vNAT;NLc,jm>, and standard deviations sσ;NLc,jm and vσ;NLc,jm within the group are calculated. The results of the calculation are stored as a database in the RAM 14 (step S108a).

Next, production of a database for a group of AD patients will be described below (processing begins at S101b). Similarly to the case of the group of normal controls, a group of AD patients including a predetermined number of persons is determined in advance by conducting the mini mental state examination (MMSE), magnetic resonance imaging (MRI) for image examination, or single photon emission computing tomography (SPECT), or the like which is an existing method intended to evaluate a cognitive function. Each of the persons has time-sequential data of a brain potential (scalp potential) thereof measured using each of twenty-one (J) sensors 2, and has the data recorded (step S102).

Similarly to the case of the group of normal controls, steps S103 to S105 are executed in order to calculate a normalized power spectrum NPS;j,m that is a first parameter of an AD patient and store the normalized power spectrum in the RAM 14. Step S106 is then executed in order to calculate a normalized power ratio NPV;j,m that is a second parameter of the AD patient and store the normalized power ratio in the RAM 14. Step S107 is executed in order to calculate sNAT;j,m and vNAT;j,m of the AD patient. Steps S101 to S107 are repeatedly performed on all the AD patents, whereby group means <sNAT;AD,jm> and <vNAT;AD,jm> for the group of AD patients and standard deviations sσ;AD,jm and vσ;AD,jm within the group are calculated. The results of the calculation are stored as a database in the RAM 14 (step S108b).

Next, processing for a measured brain potential of a subject (x) will be described below (processing begins at S101c). Similarly to the case of the group of normal controls, the sensors 2 are mounted on the subject (x), and the subject is measured in order to acquire time-sequential data of a brain potential (scalp potential) from each of the sensors 2 and record the data (step S102). Steps S103 to S105 are executed in order to calculate a normalized power spectrum

NPS;j,m that is a first parameter of the subject (x). Step S106 is executed in order to calculate a normalized power ratio NPV;j,m that is a second parameter of the subject (x). Step S107 is executed in order to calculate sNAT;x,jm and vNAT;x,jm that are a pair of markers characterizing a state of the brain function activity of the subject (x).

Next, processing of step S109 will be described below.

The group means <sNAT;NLc,jm> and <vNAT;NLc,jm> for the group of normal controls, and the standard deviations sσ;NLc,jm and vσ;NLc,jm within the group are read from the RAM 14, and are used together with sNAT;x,jm and vNAT;x,jm of the subject (x), which are obtained at step S107, to calculate sZ;j,m;x:NLc according to a formula (7) below.

[Math. 7]

$$ sZ_{j,m}^{x:NLc} \equiv \left( sNAT_{j,m}^{x} - sNAT_{j,m}^{NLc} \right) \Big/ s\sigma_{j,m}^{NLc} $$

Likewise, vZ;j,m;x:NLc is calculated according to a formula (8) below.

[Math. 8]

$$ vZ_{j,m}^{x:NLc} \equiv \left( vNAT_{j,m}^{x} - vNAT_{j,m}^{NLc} \right) \Big/ v\sigma_{j,m}^{NLc} $$

Now, the foregoing sZ;j,m;x:NLc and vZ;j,m;x:NLc are quantities representing a distance between the brain function activity of the subject (x) and the standard state of the brain function activities of the group of normal controls, and the distance may be referred to as a normalized distance or Mahalanobis distance.

In the present invention, sZ;j,m;x:NLc and vZ;j,m;x:NLc are defined as an sZ score and vZ score respectively. The vZ score is an index relevant to a coherency of a brain function activity, and the sZ score is an index relevant to a level of the brain function activity in the brain.

Now, if vZ;j,m;x:NLc>0, a brain function activity that contributes to generation of a brain potential at the position of the electrode j is in an under-synchronous state compared with those of NLc (normal controls). In contrast, if vZ;j,m;x:NLc<0, the brain function activity is in an over-synchronous state. If sZ;j,m;x:NLc>0, since the brain function activity level is larger than those of NLc, the brain function activity is in a hyperactive state. If sZ;j,m;x:NLc<0, since the brain function activity level is smaller than those of NLc, the brain function activity is in a hypoactive state. Therefore, by introducing the sZ score and vZ score, the state of the brain function activity can be classified into four states, that is, an over-synchronous and hyperactive state, an over-synchronous and hypoactive state, under-synchronous and hyperactive state, and under-synchronous and hypoactive state. The brain function activity can be characterized in more detail.

Likewise, at step S109, the group means <sNAT;AD,jm> and <vNAT;AD,jm> for the group of AD patients and the standard deviations sσ;AD,jm and vσ;NLc,jm within the group are read from the RAM 14, and used together with sNAT;x,jm and vNAT;x,jm of the subject (x), which are obtained at step S107, to calculate sZ;j,m;x:AD according to a formula (9) below.

[Math. 9]

$$ sZ_{j,m;x:AD} \equiv \left( sNAT_{j,m}^{x} - sNAT_{j,m}^{AD} \right) \Big/ s\sigma_{j,m}^{AD} $$

Likewise, vZ;j,m;x:AD is calculated according to a formula (10) below.

[Math. 10]

$$ vZ_{j,m;x:AD} \equiv \left( vNAT_{j,m}^{x} - vNAT_{j,m}^{AD} \right) \Big/ v\sigma_{j,m}^{AD} $$

The foregoing sZ;j,m;x:AD and vZ;j,m;x:AD are quantities representing a distance between the state of the brain function activity of the subject (x) and a template state of the brain function activities of the group of AD patients.

Next, processing of step S110 will be described below.

Using sZ;j,m;x:NLc and vZ;j,m;x:NLc obtained at step S109, sL;x,NLc and vL;x,NLc that represent the likelihoods of the subject (x) to the standard state of the group of normal controls are calculated according to formulae (11) and (12) respectively.

[Math. 11]

$$\exp\left\langle -(sZ_{j,m}^{x:NLc})^2 \right\rangle_{j,m}$$

[Math. 12]

$$\exp\left\langle -(vZ_{j,m}^{x:NLc})^2 \right\rangle_{j,m}$$

Likewise, sL;x,ADc and vL;x,ADc that represent the likelihoods of the subject (x) to the standard state of the group of AD patients are calculated according to formulae (13) and (14) respectively.

[Math. 13]

$$\exp\left\langle -(sZ_{j,m}^{x:AD})^2 \right\rangle_{j,m}$$

[Math. 14]

$$\exp\left\langle -(vZ_{j,m}^{x:AD})^2 \right\rangle_{j,m}$$

Next, processing of step S111 will be described below. Using the likelihoods sL;x,NLc, vL;x,NLc, sL;x,ADc, and vL;x,ADc obtained at step S110, difference likelihoods signifying to which of the standard state of the group of normal controls and the standard state of the group of AD patients the subject (x) is similar are calculated according to formulae (15) and (16) respectively.

[Math. 15]

$$sL_{x:ADc-NLc} \equiv sL_{x:ADc} - sL_{x:NLc}$$

[Math. 16]

$$vL_{x:ADc-NLc} \equiv vL_{x:ADc} - vL_{x:NLc}$$

By introducing the difference likelihoods, the standard state of the group of AD patients and the standard state of the group of normal controls can be separated from each other, and the brain function activity level of the subject (x) can be highly precisely evaluated.

The results of execution (results of computation) of the processing ending at step S111 are subjected to imaging processing, and displayed on the display unit 31 such as a CRT (step S112). The display will be described later.

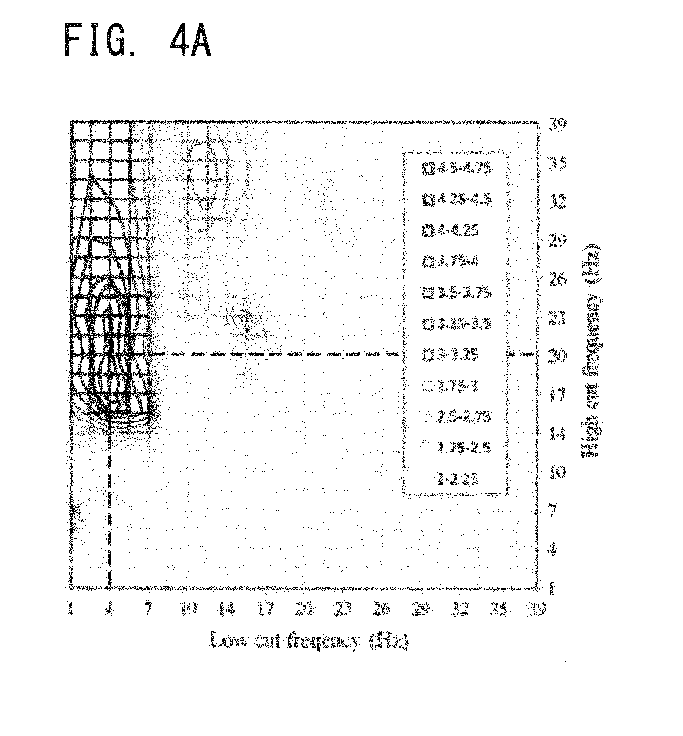

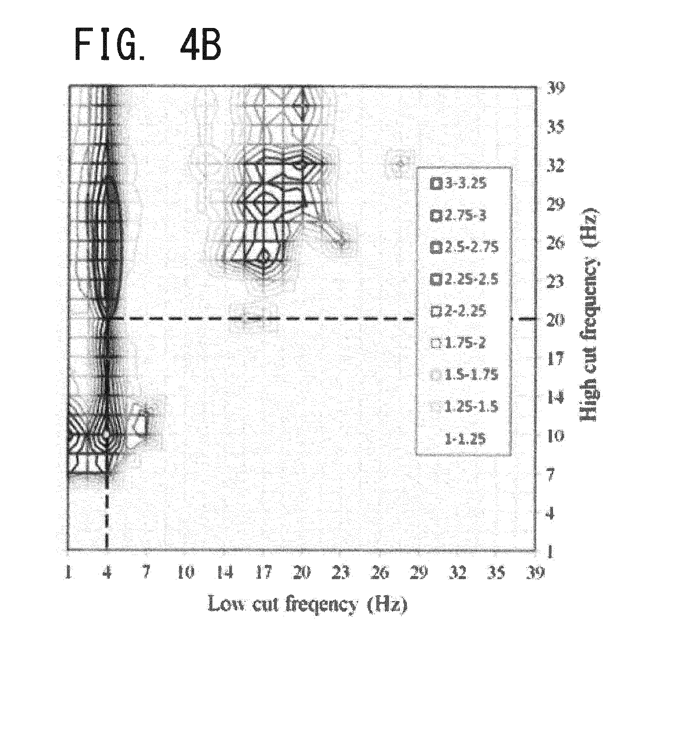

Designation of the optimal frequency band ranging from 4 Hz to 20 Hz will be described below. In general, it is almost impossible to automatically remove an unnecessary signal (artifact) such as a myoelectric signal caused by a body motion or blink occurring during brain potential measurement. Preprocessing is to merely exclude a segment in which an amplitude is equal to or higher than 100 microvolts. An optimal frequency band, that is, a frequency band in which a separation probability between an AD patient and a normal control becomes maximum was experimentally determined through trial and error. Fig. 4A and Fig. 4B show the results of an experiment. Fig. 4A and Fig. 4B are diagrams expressing t values in a t-test which represent significance of state separation. Fig. 4A is concerned with sNAT;j,m, while Fig. 4B is concerned with vNAT;j,m. The axis of abscissas indicates a lower-limit frequency, and the axis of ordinates indicates an upper-limit frequency. A frequency band in which large t values are obtained in common for the two markers sNAT;j,m and vNAT;j,m ranges from 4 Hz to 20 Hz. Therefore, in the present invention, the optimal frequency band is set to the range from 4 Hz to 20 Hz.

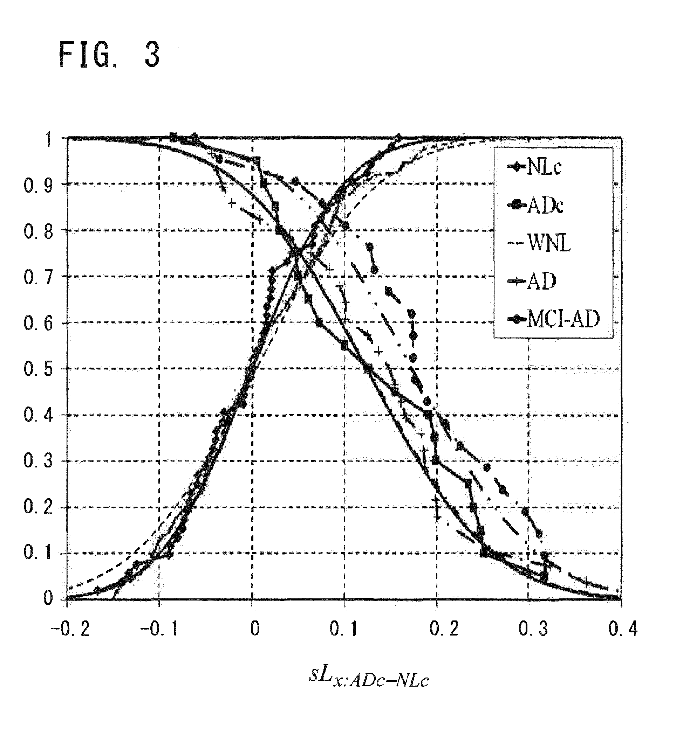

Fig. 3 is a diagram showing sensitivity/specificity curves with respect to a difference likelihood. The difference likelihood sL;x,ADc-NLc signifying to which of the template state of the group of normal controls and the template state of the group of AD patients the subject (x) is more similar is marked on the axis of abscissas, and sensitivity/specificity curves are plotted as functions of the difference likelihood. Fig. 3 demonstrates that a sensitivity or specificity of a maximum of 85 % can be obtained. In other words, an erroneous discrimination probability of discriminating AD from no disease is 15 %. Since the present invention is not accompanied by radiation exposure, if a normal state results from repeated diagnosis, a possibility of erroneous diagnosis decreases accordingly. Therefore, the present invention can be utilized for high-sensitivity screening of AD patients.

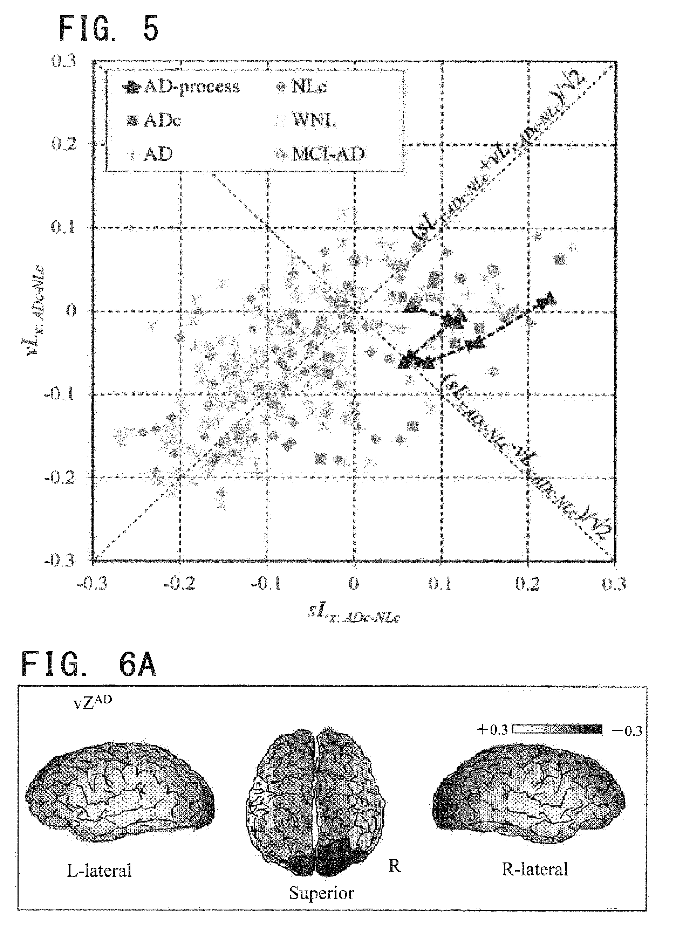

Fig. 6A to Fig. 6C are diagrams showing examples of display of NAT images and SPECT images.

In Fig. 6A, the images are displayed with vZ;j,x:AD allocated to associated positions on a standard brain surface. A bar displayed in the right upper part of the drawing is a gradation bar in which a whiter color indicates a larger positive value of the vZ score, and a blacker color indicates a larger negative value of the vZ score. A left lateral image of the standard brain surface, a superior image thereof, and a right lateral image thereof are viewed in association with the gradation bar, whereby the state of a brain function activity can be discerned. As mentioned above, if the vZ score is larger than 0, an under-synchronous state is observed. If the vZ score is smaller than 0, an over-synchronous state is observed.

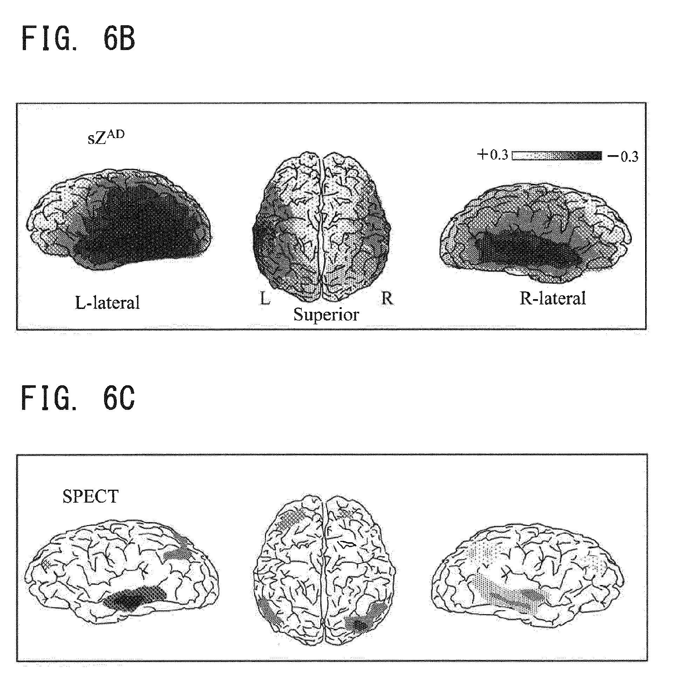

In Fig. 6B, the images are displayed with sZ;j,x:AD allocated to associated positions on a standard brain surface. A bar displayed in the right upper part of the drawing is a gradation bar in which a whiter color indicates a larger positive value of the sZ score and a blacker color indicates a larger negative value of the sZ score. The left lateral image of the standard brain surface, the superior image thereof, and the right lateral image thereof are viewed in association with the gradation bar, whereby the state of a brain function activity can be discerned. As mentioned above, if the sZ score is larger than 0, a hyperactive state is observed. If the sZ score is smaller than 0, a hypoactive state is observed.

By viewing Fig. 6A and Fig. 6B on a screen, the state of the brain function activity can be classified into four states of the over-synchronous and hyperactive state, over-synchronous and hypoactive state, under-synchronous and hyperactive state, and under-synchronous and hypoactive state.

Fig. 6C shows SPECT images. A black area in the image shows a region in which a cerebral bloodstream is decreased. In other words, regions in which the brain function activity is hypoactive and under-synchronous correspond to the cerebral bloodstream decreased regions.

Next, a case where a likelihood graph is displayed on the screen will be described below. Fig. 5 is the likelihood graph using a pair of difference likelihoods. The axis of abscissas indicates the difference likelihood $sL_{;x:ADc-NLc}$, and the axis of ordinates indicates the difference likelihood $vL_{;x:ADc-NLc}$. The difference likelihoods are obtained at step S111 mentioned in Fig. 2. Assuming that the system of coordinates is turned through 45° clockwise, a degree of severity of AD is indicated in the right upper direction over the first and second quadrants in the resultant system of coordinates, and a normal domain is spread in the left lower direction therein. Talking of a synchronous abnormality of a brain function activity and an activity level abnormality, an adverse effect of the latter one is seen greater than that of the former one in the right lower part of the resultant system of coordinates, while the adverse effect of the former one is seen greater in the left upper part thereof. A dashed line with an arrow indicates a therapeutic process of a certain AD patient. By displaying the therapeutic process while superposing it on the likelihood diagram, AD can be discovered in an early stage in course of a transition from a normal state to AD, or a therapeutic effect can be verified.

In Fig. 5, the difference likelihoods to MCI-AD are also plotted. MCI-AD expresses a group of patients who suffer from mild cognitive impairment and have a possibility of converting in AD.

Example 2

(1) Hardware configuration of a brain function activity level evaluation system

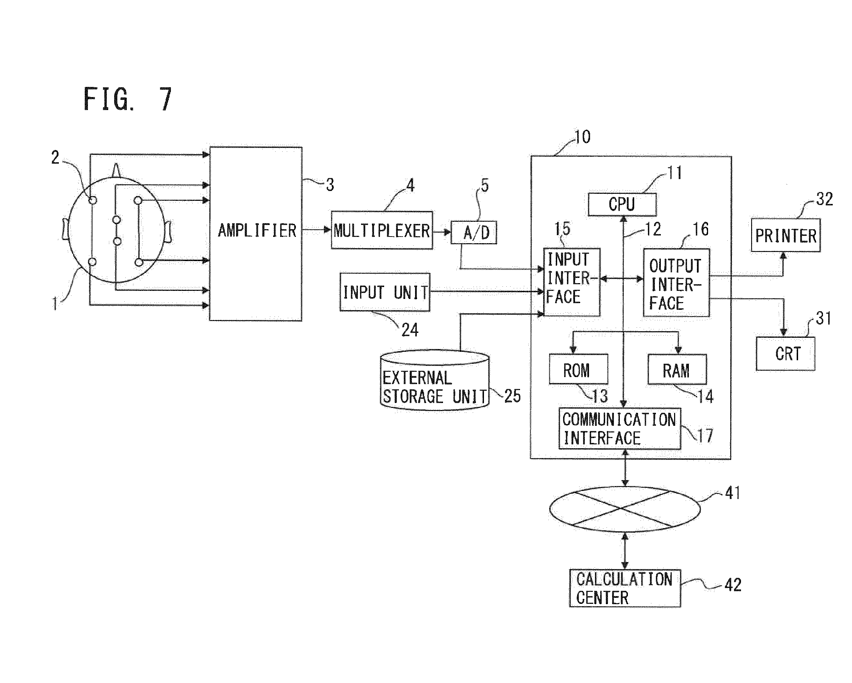

Fig. 7 is a block diagram of a brain function activity level evaluation system that is another example of the present invention.

Compared with Fig. 1, in the present example, a computer 10 acts as a data transfer terminal device installed at each of clinical sites. Measured brain potential data is transmitted to a calculation center 42, which serves as arithmetic equipment, over a communication line 41 such as the Internet via a communication interface 17 of the computer 10. The calculation center 42 performs various computations on the measured brain potential data, and transmits the results of computation or results of analysis to the computer 10 over the communication line 41. The computer 10 serving as a data transfer terminal performs image processing on the received results of computation or results of analysis, and displays the resultant data on a CRT 31, or allows an output unit such as a printer 32 to print out the resultant data. The calculation center 42 has a program and a storage medium included in a server device that is not shown. The server device or the like acquires and stores a brain potential, that is, data concerning the brain potential, stores data concerning a specific brain disease, performs various computations on the stored data concerning the brain potential, and stores the results of computation or results of analysis. By thus configuring the brain function activity level evaluation system, the calculation center computes data and manages the results of computation on a centralized manner. The computers 10 serving as data transfer terminals and being installed at respective clinical sites need to merely transmit measured brain potential data and perform image processing on the results of computation or results of analysis received from the calculation center 42. In addition to the advantage of Example 1, there is provided an advantage that loads can be dispersed.

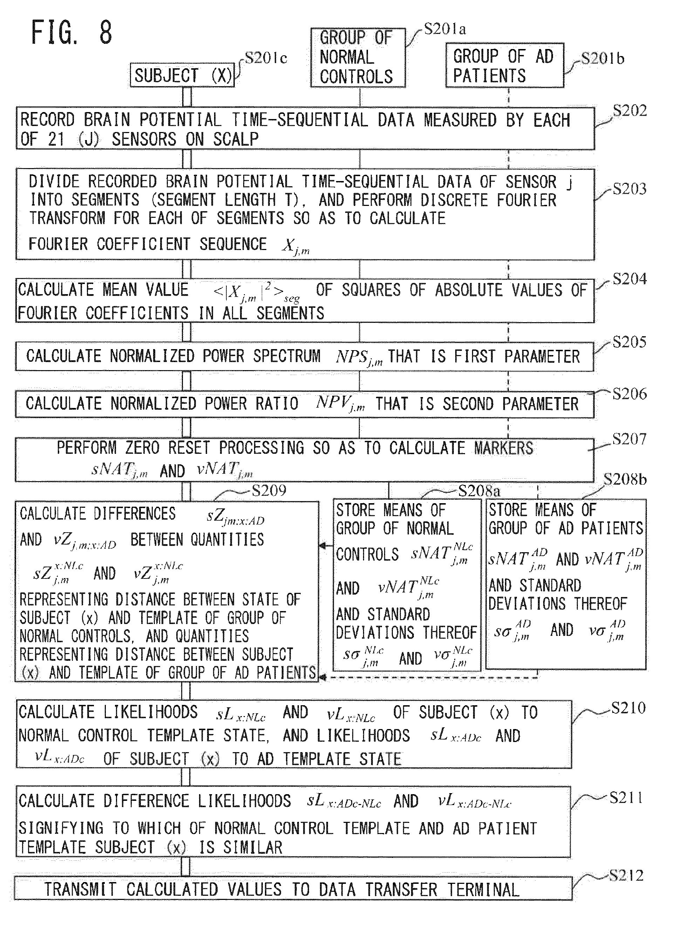

Fig. 8 is a flowchart describing processing of the brain function activity level evaluation system of the present invention. The calculation center 42 receives measured brain potential time-sequential data items of a subject (x), a group of normal controls, and a group of AD patients from the computer 10 (steps S201a, S201b, S201c, and S202). The calculation center 42 reads the program from the server device, and performs computation on the received brain potential time-sequential data items (steps S203 to S211). The contents of processing to be performed are identical to those of steps S103 to S111 mentioned in Fig. 2. An iterative description will be omitted. Calculated values that are the results of computation or results of analysis are transmitted to the data transfer terminal (step S212).

The present invention is not limited to the foregoing examples but encompasses various variants. For example, the examples have been described for a better understanding of the present invention. The present invention is not limited to a configuration including all of the described components.

**Claims**

1.   A brain function activity level evaluation device of a subject comprising:

a plurality of sensors that is mounted on the head of a subject in order to measure a brain potential of the subject; and

arithmetic means that divides a brain potential, which is outputted from each of the sensors, into segments, which have a predetermined time width, on a time base, performs discrete Fourier transform for each of the segments so as to obtain a discrete Fourier coefficient that has a frequency component, which is an integral multiple of a fundamental frequency that is an inverse number of the predetermined time width, within a predetermined frequency band, obtains a mean value of squares of absolute values of Fourier coefficients in all the segments, performs normalization using the obtained mean value of the squares of the absolute values of the Fourier coefficients so as to obtain a normalized power spectrum that is a first parameter, normalizes a product of mean values of squares of absolute values of Fourier coefficients of adjoining frequency components in all the segments using a square value of the sum of the mean values of the adjoining frequency components so as to obtain a normalized power ratio that is a second parameter, wherein the first parameter and the second parameter are used to evaluate a brain function activity level.

2.   The brain function activity level evaluation device of a subject according to Claim 1, wherein the arithmetic means obtains a first marker and a second marker by subtracting a mean value of values of the frequency component, which are derived from all the sensors, from the values of the frequency component in each of the normalized power spectrum that is the first parameter, and the normalized power ratio that is the second parameter.

3.   The brain function activity level evaluation device of a subject according to Claim 2, wherein:

the arithmetic means calculates an sZ score using the value of the first marker relevant to a subject, a mean value of the first markers obtained in advance in the same manner from a predetermined group of normal controls, and a standard deviation thereof;
the arithmetic means further calculates a vZ score using the value of the second marker relevant to the subject, a mean value of the second markers obtained in advance in the same manner from the predetermined group of normal controls, and a standard deviation thereof; and
the arithmetic means visualizes or displays the state of a brain function activity at associated positions in a brain surface image on the basis of the sZ score and vZ score.

4.   The brain function activity level evaluation device of a subject according to any one of Claims 1 to 3, wherein the predetermined frequency band ranges from 4 Hz to 20 Hz.

5.   A brain function activity level evaluation system of a subject, comprising at least:

a brain function activity measuring terminal including a plurality of sensors that is mounted on the head of a subject in order to measure a brain potential of the subject, an interface via which the brain potential outputted from each of the sensors is transmitted to outside, and an arithmetic unit; and

a calculation center connected to the brain function activity measuring terminal over a communication line, wherein

the calculation center includes arithmetic means that divides a brain potential, which is outputted from each of the sensors and sent from the brain function activity measuring terminal, into segments, which have a predetermined time width, on a time base,

performs discrete Fourier transform for each of the segments so as to obtain a Fourier coefficient that has a frequency component, which is an integral multiple of a fundamental frequency that is an inverse number of the predetermined time width, within a predetermined frequency band,

obtains a mean value of squares of absolute values of Fourier coefficients in all the segments,

normalizes the Fourier coefficients using the obtained mean value of the squares of the absolute values of the Fourier coefficients so as to obtain a normalized power spectrum that is a first marker, and

normalizes a product of mean values of squares of absolute values of Fourier coefficients of adjoining discretized frequency components using a square value of the sum of the mean values of the adjoining frequency components so as to obtain a normalized power ratio that is a second marker; and

the calculation center transmits the obtained first marker and second marker to the brain function activity measuring terminal.

6. The brain function activity level evaluation system of a subject according to Claim 5, wherein:

the arithmetic means of the calculation center obtains a first marker and a second marker by subtracting a mean value of values of the frequency component, which are derived from all the sensors, in each of the normalized power spectrum that is the first parameter, and the normalized power ratio that is the second parameter.

7. The brain function activity level evaluation system according to Claim 6, wherein:

the arithmetic means of the calculation center calculates an sZ score using the first marker relevant to a subject, a template that is a mean value of the first markers obtained in advance in the same manner from a predetermined group of normal controls, and a standard deviation thereof;

the arithmetic means further calculates a vZ score using the second marker relevant to the subject, a template that is a mean value of the second markers obtained in advance from the predetermined group of normal controls, and a standard deviation thereof; and

the brain function activity measuring terminal includes a unit that receives the calculated sZ score and vZ score over the communication line, and visualizes or displays the state of a brain activity of the subject at associated positions in a brain surface image on the basis of the sZ score and the vZ score.

8. The brain function activity level evaluation system of a subject according to Claim 7, wherein:

the arithmetic means calculates likelihoods of the subject to the template of the group of normal controls on the basis of the calculated sZ score and vZ score, calculates difference likelihoods, which signify to which of the template of the group of normal controls and the template of a group of AD patients the subject is more similar, on the basis of the calculated likelihoods, and visualizes or displays the state of the brain activity of the subject.

9. A program allowing a computer to execute a procedure of:

dividing a brain potential, which is outputted from each of a plurality of sensors that is mounted on the head of a subject in order to measure the brain potential of the subject, into segments of a predetermined time width on a time base;

performing discrete Fourier transform for each of the segments so as to obtain a discrete Fourier coefficient that has a frequency component, which is an integral multiple of a fundamental frequency that is an inverse number of the predetermined time width, within a predetermined frequency band;

obtaining a mean value of squares of absolute values of Fourier coefficients in all the segments;

performing normalization using the obtained mean value of the squares of the absolute values of the discrete Fourier coefficients so as to obtain a normalized power spectrum that is a first parameter;

normalizing a product of mean values of squares of absolute values of discrete Fourier coefficients of adjoining frequency components using a square value of the sum of the mean values of the adjoining frequency components so as to obtain a normalized power ratio that is a second parameter; and

obtaining differences by subtracting a mean value of values of the frequency component, which are derived from all the sensors, from the values of the frequency component in each of the normalized power spectrum that is the first parameter, and the normalized power ratio that is the second parameter, and obtaining a first marker and a second marker by removing the offset values.

**10.** A computer readable recording medium having the program set forth in Claim 9 recorded therein.

# FIG. 1

EP 2 965 690 A1

# FIG. 2

```
                      ┌─────────────┐
              S101c   │  GROUP OF   │ S101a  ┌──────────────┐
┌──────────────┐      │   NORMAL    │        │  GROUP OF    │  S101b
│ SUBJECT (X)  │      │  CONTROLS   │        │ AD PATIENTS  │
└──────────────┘      └─────────────┘        └──────────────┘
```

RECORD BRAIN POTENTIAL TIME-SEQUENTIAL DATA
MEASURED BY EACH OF 21 (J) SENSORS ON SCALP — S102

DIVIDE RECORDED BRAIN POTENTIAL TIME-SEQUENTIAL DATA OF SENSOR j
INTO SEGMENTS (SEGMENT LENGTH T), AND PERFORM DISCRETE FOURIER
TRANSFORM FOR EACH OF SEGMENTS SO AS TO CALCULATE FOURIER
COEFFICIENT SEQUENCE $X_{j,m}$ — S103

CALCULATE MEAN VALUE $<|X_{j,m}|^2>_{seg}$ OF SQUARES OF ABSOLUTE
VALUES OF FOURIER COEFFICIENTS IN ALL SEGMENTS — S104

CALCULATE NORMALIZED POWER SPECTRUM $NPS_{j,m}$
THAT IS FIRST MARKER — S105

CALCULATE NORMALIZED POWER RATIO $NPV_{j,m}$
THAT IS SECOND MARKER — S106

PERFORM ZERO RESET PROCESSING SO AS TO
CALCULATE $sNAT_{j,m}$ AND $vNAT_{j,m}$ — S107

CALCULATE QUANTITIES — S109
$sZ_{j,m}^{x:NLc}$ AND $vZ_{j,m}^{x:NLc}$
REPRESENTING DISTANCE BETWEEN
SUBJECT (x) AND GROUP OF NORMAL
CONTROLS, AND QUANTITIES
$sZ_{j,m}^{x:AD}$ AND $vZ_{j,m}^{x:AD}$
REPRESENTING DISTANCE BETWEEN
SUBJECT (x) AND GROUP OF AD PATIENTS

STORE MEANS OF — S108a
GROUP OF NORMAL
CONTROLS
$sNAT_{j,m}^{NLc}$ AND $vNAT_{j,m}^{NLc}$
AND STANDARD
DEVIATIONS THEREOF
$s\sigma_{j,m}^{NLc}$ AND $v\sigma_{j,m}^{NLc}$

STORE MEANS OF — S108b
GROUP OF
AD PATIENTS
$sNAT_{j,m}^{AD}$ AND $vNAT_{j,m}^{AD}$
AND STANDARD
DEVIATIONS THEREOF
$s\sigma_{j,m}^{AD}$ AND $v\sigma_{j,m}^{AD}$

CALCULATE LIKELIHOODS $sL_{x:NLc}$ AND $vL_{x:NLc}$ OF SUBJECT (x) TO
GROUP OF NORMAL CONTROLS, AND CALCULATE LIKELIHOODS $sL_{x:ADc}$ AND
$vL_{x:ADc}$ OF SUBJECT (x) TO GROUP OF AD PATIENTS — S110

CALCULATE DIFFERENCE LIKELIHOODS $vL_{x:ADc-NLc}$ AND $sL_{x:ADc-NLc}$
SIGNIFYING TO WHICH OF GROUP OF NORMAL CONTROLS AND GROUP
OF AD PATIENTS SUBJECT (x) IS SIMILAR — S111

PERFORM IMAGING PROCESSING AND DISPLAY IMAGES ON SCREEN — S112

# FIG. 3

$sL_{x:ADc-NLc}$

Legend:
- NLc
- ADc
- WNL
- AD
- MCI-AD

# FIG. 4A

# FIG. 4B

# FIG. 5

# FIG. 6A

## FIG. 6B

## FIG. 6C

FIG. 7

# FIG. 8

$\boxed{\text{SUBJECT (X)}}$ /S201c  $\boxed{\begin{array}{l}\text{GROUP OF}\\ \text{NORMAL}\\ \text{CONTROLS}\end{array}}$ /S201a  $\boxed{\begin{array}{l}\text{GROUP OF AD}\\ \text{PATIENTS}\end{array}}$ S201b

| |
|---|
| RECORD BRAIN POTENTIAL TIME-SEQUENTIAL DATA MEASURED BY EACH OF 21 (J) SENSORS ON SCALP |

S202

| |
|---|
| DIVIDE RECORDED BRAIN POTENTIAL TIME-SEQUENTIAL DATA OF SENSOR j INTO SEGMENTS (SEGMENT LENGTH T), AND PERFORM DISCRETE FOURIER TRANSFORM FOR EACH OF SEGMENTS SO AS TO CALCULATE FOURIER COEFFICIENT SEQUENCE $X_{j,m}$ |

S203

| |
|---|
| CALCULATE MEAN VALUE $<\lvert X_{j,m}\rvert^2>_{seg}$ OF SQUARES OF ABSOLUTE VALUES OF FOURIER COEFFICIENTS IN ALL SEGMENTS |

S204

| |
|---|
| CALCULATE NORMALIZED POWER SPECTRUM $NPS_{j,m}$ THAT IS FIRST PARAMETER |

S205

| |
|---|
| CALCULATE NORMALIZED POWER RATIO $NPV_{j,m}$ THAT IS SECOND PARAMETER |

S206

| |
|---|
| PERFORM ZERO RESET PROCESSING SO AS TO CALCULATE MARKERS $sNAT_{j,m}$ AND $vNAT_{j,m}$ |

S207

S209

| CALCULATE DIFFERENCES $sZ_{jm;x:AD}$ AND $vZ_{j,m;x:AD}$ BETWEEN QUANTITIES $sZ_{j,m}^{x:NLc}$ AND $vZ_{j,m}^{x:NLc}$ REPRESENTING DISTANCE BETWEEN STATE OF SUBJECT (x) AND TEMPLATE OF GROUP OF NORMAL CONTROLS, AND QUANTITIES REPRESENTING DISTANCE BETWEEN SUBJECT (x) AND TEMPLATE OF GROUP OF AD PATIENTS |
|---|

S208a

| STORE MEANS OF GROUP OF NORMAL CONTROLS $sNAT_{j,m}^{NLc}$ AND $vNAT_{j,m}^{NLc}$ AND STANDARD DEVIATIONS THEREOF $s\sigma_{j,m}^{NLc}$ AND $v\sigma_{j,m}^{NLc}$ |
|---|

S208b

| STORE MEANS OF GROUP OF AD PATIENTS $sNAT_{j,m}^{AD}$ AND $vNAT_{j,m}^{AD}$ AND STANDARD DEVIATIONS THEREOF $s\sigma_{j,m}^{AD}$ AND $v\sigma_{j,m}^{AD}$ |
|---|

| |
|---|
| CALCULATE LIKELIHOODS $sL_{x:NLc}$ AND $vL_{x:NLc}$ OF SUBJECT (x) TO NORMAL CONTROL TEMPLATE STATE, AND LIKELIHOODS $sL_{x:ADc}$ AND $vL_{x:ADc}$ OF SUBJECT (x) TO AD TEMPLATE STATE |

S210

| |
|---|
| CALCULATE DIFFERENCE LIKELIHOODS $sL_{x:ADc-NLc}$ AND $vL_{x:ADc-NLc}$ SIGNIFYING TO WHICH OF NORMAL CONTROL TEMPLATE AND AD PATIENT TEMPLATE SUBJECT (x) IS SIMILAR |

S211

| |
|---|
| TRANSMIT CALCULATED VALUES TO DATA TRANSFER TERMINAL |

S212

| **INTERNATIONAL SEARCH REPORT** | International application No. |
| | PCT/JP2014/055230 |

A. CLASSIFICATION OF SUBJECT MATTER
*A61B5/0476(2006.01)i*

According to International Patent Classification (IPC) or to both national classification and IPC

B. FIELDS SEARCHED

Minimum documentation searched (classification system followed by classification symbols)
A61B5/0476-5/0484

Documentation searched other than minimum documentation to the extent that such documents are included in the fields searched
Jitsuyo Shinan Koho        1922-1996   Jitsuyo Shinan Toroku Koho   1996-2014
Kokai Jitsuyo Shinan Koho   1971-2014   Toroku Jitsuyo Shinan Koho   1994-2014

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| A | JP 5118230 B2  (Brain Functions Laboratory, Inc.), 16 January 2013 (16.01.2013), entire text; all drawings (Family: none) | 1-10 |
| A | JP 4145344 B1  (Brain Functions Laboratory, Inc.), 03 September 2008 (03.09.2008), entire text; all drawings & US 2009/0105603 A1    & EP 2050388 A1 & DE 602008006324 D | 1-10 |

| ☒ Further documents are listed in the continuation of Box C. | ☐ See patent family annex. |

* Special categories of cited documents:
"A" document defining the general state of the art which is not considered    to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| 04 April, 2014 (04.04.14) | 15 April, 2014 (15.04.14) |

| Name and mailing address of the ISA/ | Authorized officer |
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2014/055230 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | Taishi UEDA, Toshimitsu MUSHA, Toru YAGI, "Research on the Characteristics of Alzheimer's Disease Using EEG", IEEJ Transactions on Electronics, Information and Systems, 01 October 2010 (01.10.2010), vol.130, no.10, pages 1827 to 1832 | 1-10 |

Form PCT/ISA/210 (continuation of second sheet) (July 2009)

**EP 2 965 690 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4145344 B
- JP 5118230 B